(19) Europäisches Patentamt
European Patent Office
Office européen des brevets

(11) Numéro de publication : **0 415 850 B1**

(12) # FASCICULE DE BREVET EUROPEEN

(45) Date de publication du fascicule du brevet : **12.01.94 Bulletin 94/02**

(51) Int. Cl.[5] : **C07D 333/38**, A61K 31/38

(21) Numéro de dépôt : **90402401.5**

(22) Date de dépôt : **31.08.90**

(54) **Sels de métaux bivalents de l'acide N, N-di(carboxyméthyl)amino-2 cyano-3 carboxyméthyl-4 carboxy-5 thiophène,leur procédé de préparation et les compositions pharmaceutiques les renfermant.**

(30) Priorité : **01.09.89 FR 8911475**

(43) Date de publication de la demande : **06.03.91 Bulletin 91/10**

(45) Mention de la délivrance du brevet : **12.01.94 Bulletin 94/02**

(84) Etats contractants désignés : **AT BE CH DE DK ES FR GB GR IT LI LU NL SE**

(56) Documents cités :
**BULLETIN DE LA SOCIETE CHIMIQUE DE FRANCE, vol. 7-8, partie 2, 1975, pages 1786-1792, Masson; M. WIERZBICKI et al.: "Réactivité des amino-2 thiophènes. Application à la synthèse de quelques thiéno[2,3-b]pyrroles"**

(73) Titulaire : **ADIR ET COMPAGNIE
1 rue Carle Hébert
F-92415 Courbevoie Cédex (FR)**

(72) Inventeur : **Wierzbicki, Michel
32 rue Lucien Voilin
F-92800 Puteaux (FR)**
Inventeur : **Bonnet, Jacqueline
194 bld Bineau
F-92200 Neuilly sur Seine (FR)**
Inventeur : **Brisset, Martine
6 Place de l'Ancienne Boucherie
F-14000 Caen (FR)**
Inventeur : **Tsouderos, Yannis
80 Elysée II
F-78170 La Celle Saint Cloud (FR)**

(74) Mandataire : **Reverbori, Marcelle
Adir et Compagnie, 1, rue Carle Hébert
F-92415 Courbevoie Cédex (FR)**

**EP 0 415 850 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention a pour objet de nouveaux sels de métaux bivalents de l'acide N, N-di(carboxyméthyl)amino-2 cyano-3 carboxyméthyl-4 carboxy-5 thiophène, leur procédé de préparation et les compositions pharmaceutiques les refermant.

Elle concerne donc les sels de métaux bivalents de l'acide N, N-di(carboxyméthyl)amino-2 cyano-3 carboxyméthyl-4 carboxy-5 thiophène de formule générale I :

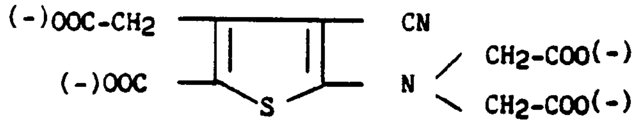

2 M (++)

(I)

dans laquelle M représente un métal bivalent tel que strontium, calcium ou magnésium.

La présente invention a également pour objet le procédé de préparation des sels de formule générale I caractérisé en ce que : l'on utilise comme matière première le tétraester de formule II :

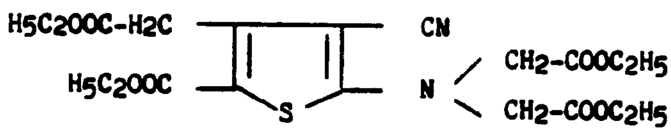

(II)

lequel est :

a) soit chauffé à reflux, en présence de soude, en milieu hydroalcoolique puis hydrolysé en milieu acide pour donner l'acide de formule III :

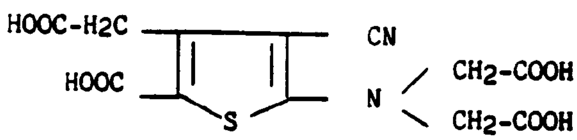

(III)

que l'on fait réagir, en milieu aqueux, avec l'hydroxyde de formule générale (IV) :

$$M(OH)_2 \qquad (IV)$$

dans laquelle M a la signification précédemment définie;

b) soit chauffé à reflux dans un mélange 50/50 en volume d'une solution normale de soude et d'éthanol afin d'obtenir, après distillation des solvants, le sel tétrasodique de formule V

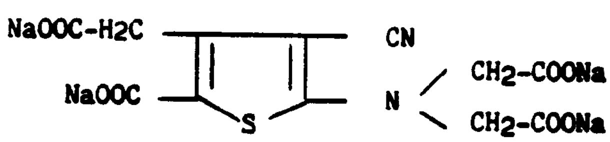

(V)

que l'on traite par une solution aqueuse de chlorure de formule VI :

$$M\,Cl_2 \qquad (VI)$$

dans laquelle M a la signification précédemment définie ;

c) soit chauffé à reflux, en milieu hydroalcoolique, avec l'hydroxyde de formule générale IV précédemment définie.

Ces trois méthodes ne sont en fait que les variantes d'un même procédé qui consiste à préparer les sels de formule I à partir du tétraester de formule II soit directement (cas C) soit en isolant intermédiairement le tétracide de formule III (cas a) ou le sel tétrasodique de formule V (cas b).

La matière première de formule II est décrite dans la littérature (cf. M. Wierzbicki et al. Bull. Soc. Chim. (1975) pages 1786-92).

L'acide N, N-di(carboxyméthyl)amino-2 cyano-3 carboxyméthyl-4 carboxy-5 thiophène de formule III est

un produit nouveau qui peut être utilisé comme matière première dans l'industrie chimique et pharmaceutique, notamment dans la synthèse des sels de métaux bivalents de formule I. Il est donc, à ce titre, inclus dans la présente invention.

Les sels de métaux bivalents de formule I possèdent des propriétés pharmacologiques et thérapeutiques intéressantes, notamment des propriétés anti-ostéoporotiques remarquables, qui, de ce fait, permettent leur utilisation comme médicament notamment dans le traitement des maladies osseuses. Ils peuvent également être utilisés dans le traitement du vieillissement cutané et vasculaire, des affections hépatiques et des affections dentaires.

Il est connu, de l'état antérieur de la technique que certains sels de métaux bivalents sont utilisables en thérapeutique notamment dans le traitement des maladies osseuses. Par exemple, certaines publications de la littérature, notamment Gastineau, Poc. Staff. Meetings Mayo Clinic 35, 105-11 (1960) ; Skoryna, Can. Med. Assoc. 125 (7), 702-12 (1981), Skoryna, Trace Subst. Environ. Health 18, 3-23 (1984) - font état de l'activité des lactate, gluconate et carbonate de strontium dans le traitement de l'ostéoporose.

Les sels de métaux bivalents de la présente invention, outre le fait d'être nouveau vis à vis des sels préalablement cités, présentent par rapport à ces derniers, des avantages surprenants, notamment une meilleure biodisponibilité, ce qui permet d'administrer des doses chimiques moindres lors du traitement de l'ostéoporose.

La présente invention a également pour objet les compositions pharmaceutiques contenant comme principe actif un des sels de formule I, mélangé ou associé à un excipient pharmaceutique approprié, comme par exemple, l'eau distillée, le glucose, le lactose, l'amidon, le talc, l'éthyl cellulose, le stéarate de magnésium ou le beurre de cacao.

Les compositions pharmaceutiques ainsi obtenues se présentent généralement sous forme dosée et peuvent contenir de 200 à 300 mg de principe actif. Elles peuvent revêtir la forme de comprimés, dragées, gélules, solutions buvables, solutions injectables ou suppositoires, et être, selon les cas, administrées par voie orale, rectale ou parentérale à la dose de 200 à 300 mg 2 à 4 fois par jour.

Les exemples suivants illustrent l'invention.

Exemple 1 :

Synthèse du sel distrontique de l'acide N, N-di(carboxyméthyl)amino-2 cyano-3 carboxyméthyl-4 carboxy-5 thiophène :

a) première méthode :

1 mole (454,5 g) de tétraester éthylique de l'acide N, N-di(carboxyméthyl)amino-2 cyano-3 carboxyméthyl-4 carboxy-5 thiophène est portée à reflux dans un mélange de 4 litres d'éthanol, 5 l d'une solution normale de soude et 4 litres d'eau, pendant environ 3 heures.

L'alcool est ensuite distillé ; le milieu aqueux, refroidi est additionné à 1.250 ml de solution 4N d'HCl, et le tout-est précipité sur 30 litres d'acétone. On filtre le chlorure de sodium, distille l'acétone et traite la solution aqueuse par de la résine sulfonique régénérée en cycle ($H^{(+)}$ jusqu'à disparition du sodium dans la solution. On évapore à sec et recristallise le résidu, dans l'éther éthylique puis le tétrahydrofuranne ou l'acétone. On obtient ainsi l'acide pur, éventuellement cristallisé avec le solvant (26 % de solvant dans le cas du tétrahydrofuranne et 11 % dans le cas de l'acétone) et correspondant à 240 g d'acide pur (rendement : 70 %).

On additionne dans 660 ml d'eau, l'équivalent solvaté de 34,2 g (0,1 mole) d'acide. On distille le solvant organique sous vide à 20°C. A la solution aqueuse restante on ajoute 53,14 g d'hydroxyde de strontium, $8H_2O$. On filtre, laisse cristalliser pendant 24 à 48 heures, puis filtre.

On obtient ainsi l'octahydrate du sel distrontique de l'acide N, N-di(carboxyméthyl) amino-2 cyano-3 carboxyméthyl-4 carboxy-5 thiophène, lequel, par séchage sous courant d'air sec donne l'heptahydrate.

Après séchage de ce dernier, sous pression réduite (10 mm) à 55°C, on obtient le téthrahydrate correspondant.

L'acide libre peut également être recristallisé directement avec l'éther éthylique. Dans ce cas, il cristallise avec 4 % d'éther.

Les caractéristiques physiques des produits ainsi préparés sont :

- Acide (éther) :

IR  ν (OH) : 2000 et 3700 $cm^{-1}$
ν (CN) : 2220 $cm^{-1}$
ν (CO) : 1680 et 1720 $cm^{-1}$

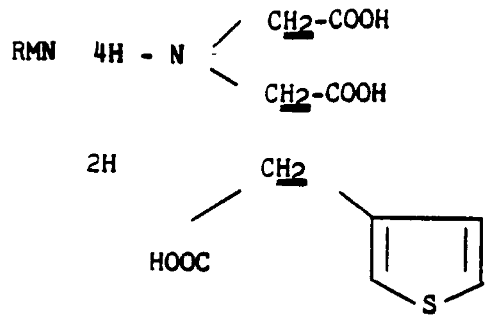

δ : 4,4 ppm

δ : 3,9 ppm

- octahydrate :

IR ν (CN) : 2206 $cm^{-1}$

- heptahydrate :

IR ν (CN) : 2210 $cm^{-1}$
ν ($COO^-$) : 1500 - 1700 $cm^{-1}$

- tetrahydrate :

IR ν (CN) : 2200 et 2220 $cm^{-1}$
ν ($COO^-$) : centrée à 1580 $cm^{-1}$
RMN 4H - δ : 4,4 ppm
2H - δ : 3,9 ppm

d) Deuxième méthode

Une mole de tétraester éthylique de l'acide N, N-di(carboxmméthyl)amino-2 cyano-3 carboxyméthyl-4 carboxy-5 thiophène est portée à reflux dans un mélange de 4 litres de soude normale et 4 litres d'éthanol pendant environ 4 heures.

On vérifie par RMN la disparition des groupements esters. Dès qu'elle est totale, on distille l'éthanol et la majorité de l'eau (jusqu'à un volume d'un litre) sous vide, au bain-marie. L'huile obtenue est précipitée dans 20 litres d'éthanol. Le sel de sodium obtenu est filtré puis séché sous vide à 50°C.

Une mole de sel tétrasodique est dissoute dans 4 litres d'eau. La solution filtrée est ajoutée à une solution de 2 moles de chlorure de strontium dans 4 litres d'eau. Le tout est homogénéisé rapidement puis laissé au repos 24 heures.

On filtre le sel formé, qui est le sel distrontique, sous forme octahydrate, de l'acide N, N-di(carboxyméthyl)amino-2 cyano-3 carboxyméthyl-4 carboxy-5 thiophène.

c) Troisième méthode

On porte à reflux pendant environ une heure, un mélange de 1 mole de tétraester éthylique de l'acide N, N-di(carboxyméthyl)amino-2 cyano-3 carboxyméthyl-4 carboxy-5 thiophène, deux moles d'hydroxyde de strontium, 4 litres d'eau et 4 litres d'éthanol.

On distille ensuite l'éthanol, porte la solution aqueuse à 100°C, filtre à chaud, lave le résidu avec quelques dizaines de ml d'eau et filtre l'octahydrate du sel distrontique de l'acide N, N-di(carboxyméthyl)amino-2 cyano-3 carboxyméthyl-4 carboxy-5 thiophène, ainsi obtenu.

En opérant, comme dans la première méthode, les heptahydrate et tétrahydrate du sel distrontique de l'acide N, N-di(carboxyméthyl) amino-2 cyano-3 carboxyméthyl-4 carboxy-5 thiophène ont été préparés à partir de l'octahydrate obtenu selon les deuxième et troisième méthodes ci-dessus

Exemple 2

Etude pharmacologique

a) Propriétés anti-résorbantes

Des propriétés anti-résorbantes osseuses ont été mises en évidence sur les calvaria de souris selon un modèle inspiré de la méthode décrite par REYNOLDS et DINGLE - A sensitive in vitro method for studying the

induction and inhibition of bone resorption, Calc. Tiss. Res., 4, 339-349 (1970).

En bref, le relargage du $Ca^{45}$ préalablement incorporé à l'os par injection sous-cutanée à l'animal, est mesuré au bout de 48 heures de culture des calvaria en présence ou non du principe actif.

Ainsi, en utilisant le sel distrontique heptahydrate de l'acide N, N-di(carboxyméthyl)amino-2 cyano-3 carboxyméthyl-4 carboxy-5 thiophène, préparé selon l'exemple 1, dans l'évaluation de la résorption osseuse sur calvaria de souris, on a obtenu les résultats suivants :

| **Concentration M** | | **n** | **Témoin** | **Traité** | **Signification statistique** | **% variation** |
|---|---|---|---|---|---|---|
| **Sel** | **Sr** | | | | | |
| $5.10^{-5}$ | $10^{-4}$ | 15 | 11,22 ±0,33 | 11,00 ±0,26 | NS | - 1,4 % ±2,3 |
| $10^{-4}$ | $2.10^{-4}$ | 36 | 11,63 ±0,26 | 10,96 ±0,20 | ** | - 5,0 % ±1,6 |
| $5.10^{-4}$ | $10^{-3}$ | 13 | 13,25 ±0,26 | 10,95 ±0,21 | *** | - 17,1 % ±1,7 |
| $8.10^{-4}$ | $1,6.10^{-3}$ | 14 | 11,86 ±0,44 | 9,35 ±0,31 | *** | - 20,9 % ±1,6 |
| $10^{-3}$ | $2.10^{-3}$ | 14 | 14,38 ±0,39 | 10,30 ±0,34 | *** | - 28,1 % ±2,0 |

Sel = sel distrontique heptahydrate de l'acide N, N-di(carboxyméthyl)amino-2 cyano-3 carboxyméthyl-4 carboxy-5 thiophène

n = nombre de calvaria/lot

moyenne ± erreur standard

comparaison statistique par test de t pour séries appariées NS : P>0,05 ; ** : P<0,01 ; *** : P<0,001

Ces résultats se traduisent par la courbe ci-après :

ACTIVITE DUDIT SEL SUR LA RESORPTION OSSEUSE BASALE IN VITRO

(calvaria de souris)

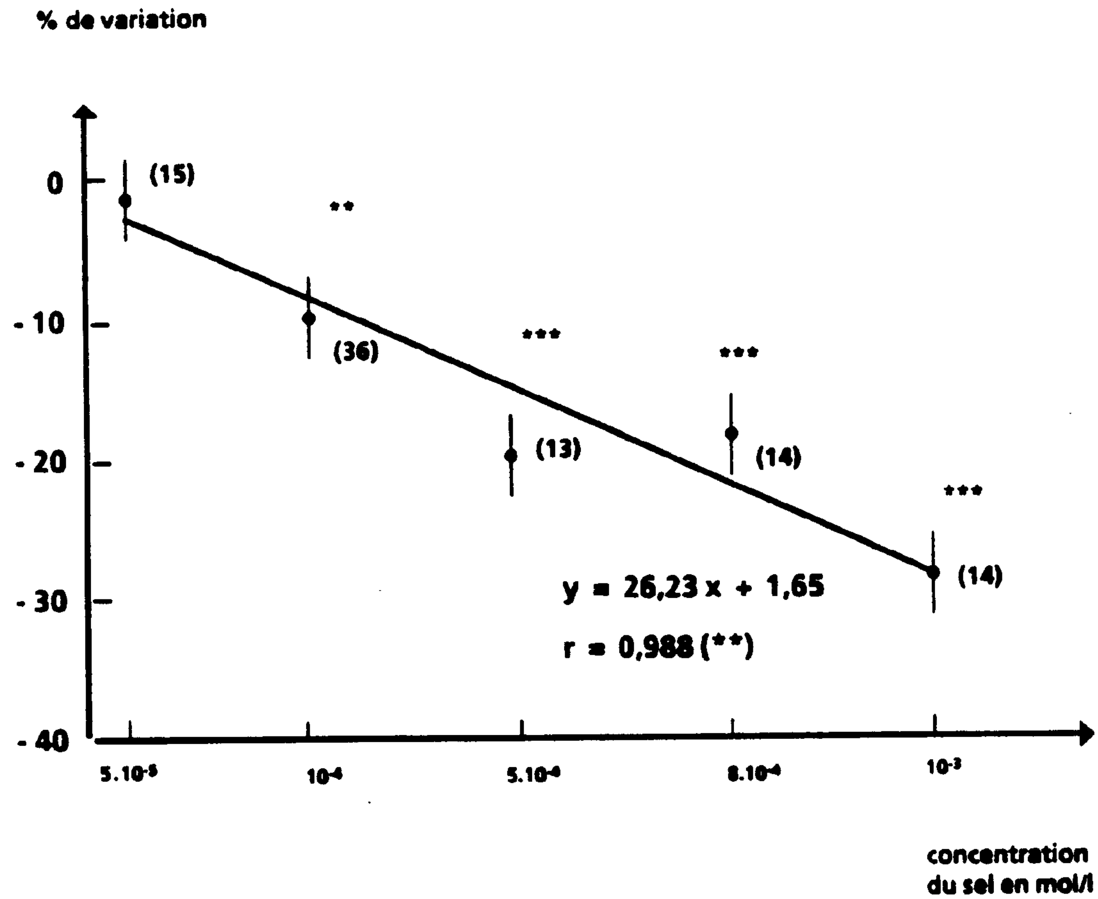


( ) nombre de calvaria étudiés

moyenne ± erreur standard

comparaison statistique par test de t pour séries appariées

NS : P>0,01 ; ** : P<0,01 ; *** : P<0,001

b) Biodisponibilité

Elle résulte de l'étude de la cinétique sérique du strontium après administration orale chez le rat du sel distrontique heptahydrate de l'acide N, N-di(carboxyméthyl)amino-2 cyano-3 carboxyméthyl-4 carboxy-5 thiophène, préparé selon l'exemple 1.

Après administration orale unique de 50 mg/kg (en équivalent strontium) de sel distrontique heptahydrate de l'acide N, N-di(carboxyméthyl)amino-2 cyano-3 carboxyméthyl-4 carboxy-5 thiophène, le strontium est absorbé avec une biodisponibilité absolue de 36,3 % ; cette dernière étant calculée à partir de celle (admise comme étant de 100 %) du chlorure de strontium par voie IV.

## Revendications

**Revendications pour les Etats contractants suivants : AT, BE, CH, DE, DK, FR, GB, IT, LI, LU, NL, SE**

1. Les sels de métaux bivalents de l'acide N, N-di(carboxyméthyl)amino-2 cyano-3 carboxyméthyl-4 carboxy-5 thiophène de formule générale I :

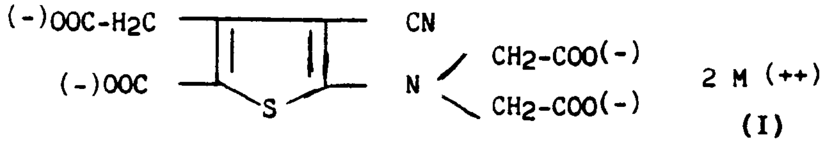

(I)

dans laquelle M représente un métal bivalent tel que strontium, calcium ou magnésium.

2. Le sel distrontique de l'acide N, N-di(carboxyméthyl)amino-2 cyano-3 carboxyméthyl-4 carboxy-5 thiophène sous forme d'octahydrate, d'heptahydrate et de tétrahydrate.

3. L'acide N, N-di(carboxyméthyl)amino-2 cyano-3 carboxyméthyl-4 carboxy-5 thiophène.

4. Le procédé de préparation des sels de métaux bivalents de la revendication 1, caractérisé en ce que l'on utilise comme matière première le tétraester de formule II :

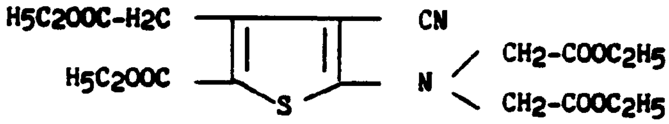

(II)

lequel est :

a) soit chauffé à reflux, en présence de soude, en milieu hydroalcoolique puis hydrolysé en milieu acide pour donner l'acide de formule III :

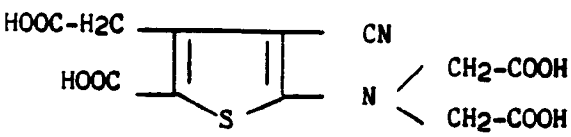

(III)

que l'on fait réagir, en milieu aqueux, avec l'hydroxyde de formule générale (IV) :

$$M(OH)_2 \qquad (IV)$$

dans laquelle M a la signification définie dans la revendication 1 ;

b) soit chauffé à reflux dans un mélange 50/50 en volume d'une solution normale de soude et d'éthanol afin d'obtenir, après distillation des solvants, le sel tétrasodique de formule V

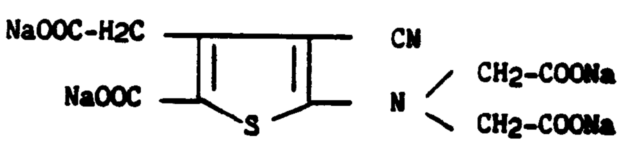

(V)

que l'on traite par une solution aqueuse de chlorure de formule VI :

$$M\,Cl_2 \qquad (VI)$$

dans laquelle M a la signification définie dans la revendication 1 ;

c) soit chauffé à reflux, en milieu hydroalcoolique, avec l'hydroxyde de formule générale IV précédemment définie.

**5.** Les compositions pharmaceutiques contenant, comme principe actif, un sel de la revendication 1, avec des excipients pharmaceutiques appropriés.

**6.** Les compositions pharmaceutiques contenant comme principe actif, le sel de la revendication 2, avec des excipients pharmaceutiques appropriés.

**7.** Les compositions pharmaceutiques selon les revendications 5 et 6, présentées sous une forme convenant notamment pour le traitement des maladies osseuses, du vieillissement cutané et vasculaire, des affections hépatiques et des affections dentaires.

**Revendications pour l'Etat contractant suivant : GR**

**1.** Le procédé de préparation des sels de métaux bivalents de l'acide N, N-di(carboxyméthyl)amino-2 cyano-3 carboxyméthyl-4 carboxy-5 thiophène de formule générale I :

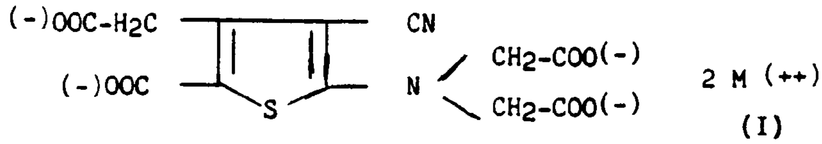

(I)

dans laquelle M représente un métal bivalent tel que strontium, calcium ou magnésium, caractérisé en ce que l'on utilise comme matière première le tétraester de formule II :

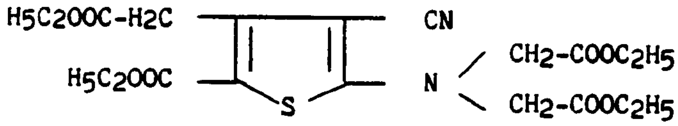

(II)

lequel est :

a) soit chauffé à reflux, en présence de soude, en milieu hydroalcoolique puis hydrolysé en milieu acide pour donner l'acide de formule III :

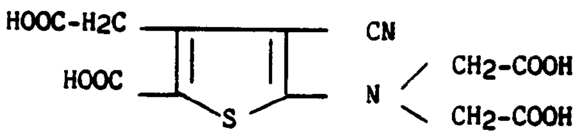

(III)

que l'on fait réagir, en milieu aqueux, avec l'hydroxyde de formule générale (IV) :

$$M(OH)_2 \qquad (IV)$$

dans laquelle M a la signification définie précédemment ;

b) soit chauffé à reflux dans un mélange 50/50 en volume d'une solution normale de soude et d'éthanol afin d'obtenir, après distillation des solvants, le sel tétrasodique de formule V

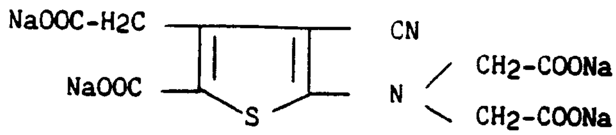

(V)

que l'on traite par une solution aqueuse de chlorure de formule VI :

$$M\,Cl_2 \qquad (VI)$$

dans laquelle M a la signification définie précédemment ;

c) soit chauffé à reflux, en milieu hydroalcoolique avec l'hydroxyde de formule générale IV précédemment définie.

2. Le procédé selon la revendication 1, pour préparer le sel distrontique de l'acide N, N-di(carboxyméthyl)amino-2 cyano-3 carboxyméthyl-4 carboxy-5 thiophéne sous forme d'octahydrate, d'heptahydrate et de tétrahydrate.

3. Le procédé de préparation de l'acide N, N-di(carboxymethyl) amino-2 cyano-3 carboxyméthyl-4 carboxy-5 thiophène caractérisé en ce que : le tétraester de formule II définie dans la revendication 1, est chauffé à reflux, en présence de soude, en milieu hydroalcoolique, puis hydrolysé en milieu acide pour donner l'acide de formule III définie dans la revendication 1, lequel acide est un produit nouveau utilisé comme intermédiaire de synthèse pour préparer, selon les revendications 1 et 2, les sels de formule I définie dans la revendication 1.

**Revendications pour l'Etat contractant suivant : ES**

1. Le procédé de préparation des sels de métaux bivalents de l'acide N, N-di(carboxyméthyl)amino-2 cyano-3 carboxyméthyl-4 carboxy-5 thiophène de formule générale I :

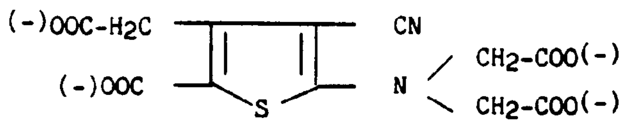

2 M (++)

(I)

dans laquelle M représente un métal bivalent tel que strontium, calcium ou magnésium, caractérisé en ce que l'on utilise comme matière première le tétraester de formule II :

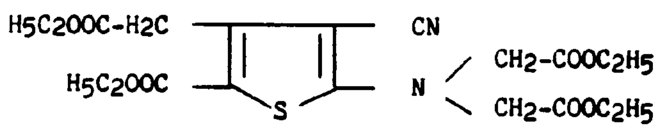

(II)

lequel est :

a) soit chauffé à reflux, en présence de soude, en milieu hydroalcoolique puis hydrolysé en milieu acide pour donner l'acide de formule III :

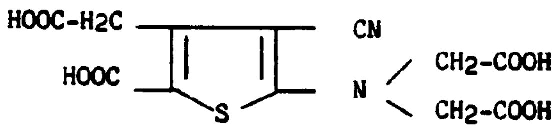

(III)

que l'on fait réagir, en milieu aqueux, avec l'hydroxyde de formule générale (IV) :

$$M(OH)_2 \qquad (IV)$$

dans laquelle M a la signification définie précédemment ;

b) soit chauffé à reflux dans un mélange 50/50 en volume d'une solution normale de soude et d'éthanol afin d'obtenir, après distillation des solvants, le sel tétrasodique de formule V

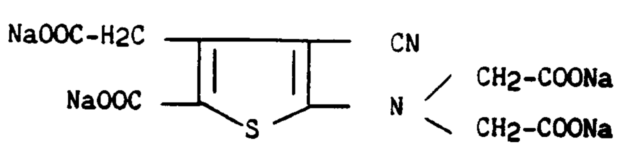

(V)

que l'on traite par une solution aqueuse de chlorure de formule VI :

$$M\,Cl_2 \qquad (VI)$$

dans laquelle M a la signification définie précédemment ;

c) soit chauffé à reflux, en milieu hydroalcoolique, avec l'hydroxyde de formule générale IV précédemment définie.

2. Le procédé selon la revendication 1, pour préparer le sel distrontique de l'acide N, N-di(carboxyméthyl)amino-2 cyano-3 carboxyméthyl-4 carboxy-5 thiophène sous forme d'octahydrate, d'heptahydrate et de tétrahydrate.

3. Le procédé de préparation de l'acide N, N-di(carboxyméthyl) amino-2 cyano-3 carboxyméthyl-4 carboxy-5 thiophène caractérisé en ce que : le tétraester de formule II définie dans la revendication 1, est chauffé à reflux, en présence de soude, en milieu hydroalcoolique, puis hydrolysé en milieu acide pour donner l'acide de formule III définie dans la revendication 1, lequel acide est un produit nouveau utilisé comme intermédiaire de synthèse pour préparer, selon les revendications 1 et 2, les sels de formule I définie dans la revendication 1.

## Patentansprüche

### Patentansprüche für folgende Vertragsstaaten : AT, BE, CH, DE, DK, FR, GB, IT, LI, LU, NL, SE

1. Salze der Säure 2-N,N-Di(carboxymethyl)-amino-3-cyano-4-carboxymethyl-5-carboxy-thiophen mit zweiwertigen Metallen der allgemeinen Formel I:

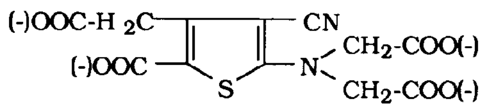

2 M (++) (I)

worin M ein zweiwertiges Metall, wie Strontium, Calcium oder Magnesium bedeutet.

2. Distrontiumsalz der Säure 2-N,N-Di(carboxymethyl)-amino-3-cyano-4-carboxymethyl-5-carboxy-thiophen in Form des Octahydrats, Heptahydrats und Tetrahydrats.

3. Die Säure 2-N,N-Di(carboxymethyl)-amino-3-cyano-4-carboxymethyl-5-carboxy-thiophen.

4. Verfahren zur Herstellung der Salze mit zweiwertigen Metallen nach Anspruch 1, **dadurch gekennzeichnet,** daß man als Ausgangsmaterial den Tetraester der Formel II:

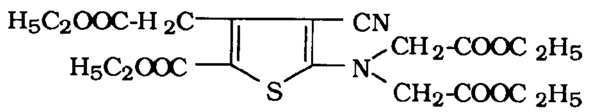

(II)

verwendet, den man

a) entweder in Gegenwart von Natriumhydroxid in wäßrig-alkoholischem Medium zum Sieden am Rückfluß erhitzt und dann in saurem Medium zur Bildung der Säure der Formel III:

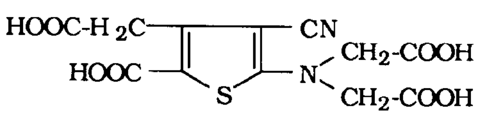

(III)

hydrolysiert, welche man in wäßrigem Medium mit dem Hydroxid der allgemeinen Formel (IV):

$$M(OH)_2 \qquad (IV)$$

in der M die in Anspruch 1 angegebenen Bedeutungen besitzt, umsetzt;

b) oder in einer 50/50 (Volumen)-Mischung aus einer 1n Natriumhydroxidlösung und Ethanol zum Sieden am Rückfluß erhitzt, so daß man nach dem Abdestillieren der Lösungsmittel das Tetranatriumsalz der Formel V

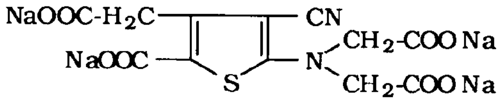

(V)

erhält, welches man in wäßriger Lösung mit dem Chlorid der Formel VI:

$MCl_2$ (VI)

in der M die in Anspruch 1 angegebenen Bedeutungen besitzt, behandelt; oder

c) in wäßrig-alkoholischem Medium mit dem oben definierten Hydroxid der allgemeinen Formel IV zum Sieden am Rückfluß erhitzt.

5. Pharmazeutische Zubereitungen enthaltend als Wirkstoff ein Salz nach Anspruch 1 zusammen mit geeigneten pharmazeutischen Trägermaterialien.

6. Pharmazeutische Zubereitungen enthaltend als Wirkstoff das Salz nach Anspruch 2 zusammen mit geeigneten pharmazeutischen Trägermaterialien.

7. Pharmazeutische Zubereitungen nach den Ansprüchen 5 und 6, die in einer Form vorliegen, die insbesondere zur Behandlung von Knochenerkrankungen, des Alterns der Haut und der Gefäße, von Lebererkrankungen und Zahnerkrankungen geeignet ist.

**Patentansprüche für folgenden Vertragsstaat : GR**

1. Verfahren zur Herstellung von Salzen der Säure 2-N,N-Di(carboxymethyl)-amino-3-cyano-4-carboxymethyl-5-carboxy-thiophen mit zweiwertigen Metallen der allgemeinen Formel I:

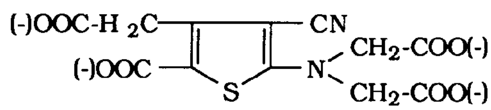

2 M (++) (I)

worin M ein zweiwertiges Metall, wie Strontium, Calcium oder Magnesium bedeutet, **dadurch gekennzeichnet,** daß man als Ausgangsmaterial den Tetraester der Formel II:

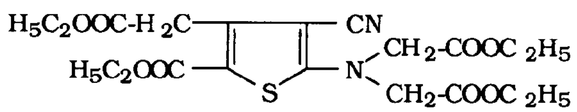

(II)

verwendet, den man

a) entweder in Gegenwart von Natriumhydroxid in wäßrig-alkoholischem Medium zum Sieden am Rückfluß erhitzt und dann in saurem Medium zur Bildung der Saure der Formel III:

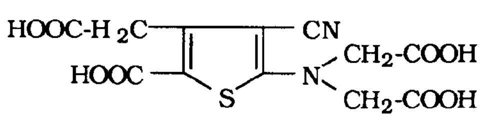

(III)

hydrolysiert, welche man in wäßrigem Medium mit dem Hydroxid der allgemeinen Formel (IV):

$M(OH)_2$ (IV)

in der M die in Anspruch 1 angegebenen Bedeutungen besitzt, umsetzt;

b) oder in einer 50/50 (Volumen)-Mischung aus einer 1n Natriumhydroxidlösung und Ethanol zum Sieden am Rückfluß erhitzt, so daß man nach dem Abdestllieren der Lösungsmittel das Tetranatriumsalz der Formel V

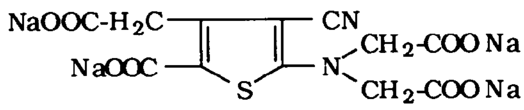

(V)

erhält, welches man in wäßriger Lösung mit dem Chlorid der Formel VI:

$$MCl_2 \qquad (VI)$$

in der M die in Anspruch 1 angegebenen Bedeutungen besitzt, behandelt; oder

c) in wäßrig-alkoholischem Medium mit dem oben definierten Hydroxid der allgemeinen Formel IV zum Sieden am Rückfluß erhitzt.

2. Verfahren nach Anspruch 1 zur Herstellung des Distrontiumsalzes der Säure 2-N,N-Di(carboxymethyl)-amino-3-cyano-4-carboxymethyl-5-carboxythiophen in Form des Octahydrats, Heptahydrats und Tetrahydrats.

3. Verfahren zur Herstellung der Säure 2-N,N-Di(carboxymethyl)-amino-3-cyano-4-carboxymethyl-5-carboxy-thiophen, **dadurch gekennzeichnet,** daß man den in Anspruch 1 definierten Tetraester der Formel I in Gegenwart von Natriumhydroxid in wäßrig alkoholischem Medium zum Sieden am Rückfluß erhitzt und dann in saurem Medium hydrolysiert zur Bildung der Säure der in Anspruch 1 definierten Formel III, welche Säure ein neues Produkt ist, das als Synthesezwischenprodukt zur Herstellung der Salze der in Anspruch 1 definierten Formel I gemäß den Ansprüchen 1 und 2 verwendet werden kann.

**Patentansprüche für folgenden Vertragsstaat : ES**

1. Verfahren zur Herstellung von Salzen der Säure 2-N,N-Di(carboxymethyl)-amino-3-cyano-4-carboxymethyl-5-carboxy-thiophen mit zweiwertigen Metallen der allgemeinen Formel I:

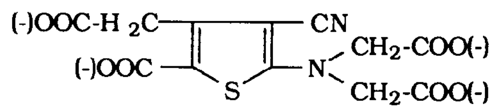

2 M (++) (I)

worin M ein zweiwertiges Metall, wie Strontium, Calcium oder Magnesium bedeutet, **dadurch gekennzeichnet,** daß man als Ausgangsmaterial den Tetraester der Formel II:

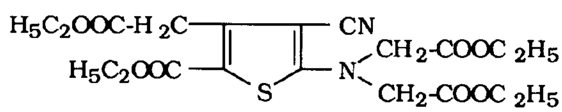

(II)

verwendet, den man

a) entweder in Gegenwart von Natriumhydroxid in wäßrig-alkoholischem Medium zum Sieden am Rückfluß erhitzt und dann in saurem Medium zur Bildung der Säure der Formel III:

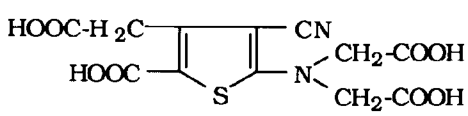

(III)

hydrolysiert, welche man in wäßrigem Medium mit dem Hydroxid der allgemeinen Formel (IV):

$$M(OH)_2 \qquad (IV)$$

in der M die in Anspruch 1 angegebenen Bedeutungen besitzt, umsetzt;

b) oder in einer 50/50 (Volumen)-Mischung aus einer 1n Natriumhydroxidlösung und Ethanol zum Sieden am Rückfluß erhitzt, so daß man nach dem Abstellieren der Lösungsmittel das Tetranatriumsalz der Formel V

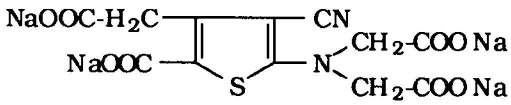

(V)

erhält, welches man in wäßriger Lösung mit dem Chlorid der Formel VI:

$$MCl_2 \qquad (VI)$$

in der M die in Anspruch 1 angegebenen Bedeutungen besitzt, behandelt; oder

c) in wäßrig-alkoholischem Medium mit dem oben definierten Hydroxid der allgemeinen Formel IV zum Sieden am Rückfluß erhitzt.

2. Verfahren nach Anspruch 1 zur Herstellung des Distrontiumsalzes der Säure 2-N,N-Di(carboxymethyl)-amino-3-cyano-4-carboxymethyl-5-carboxythiophen in Form des Octahydrats, Heptahydrats und Tetrahydrats.

3. Verfahren zur Herstellung der Säure 2-N,N-Di(carboxymethyl)-amino-3-cyano-4-carboxymethyl-5-carboxy-thiophen, **dadurch gekennzeichnet,** daß man den in Anspruch 1 definierten Tetraester der Formel I in Gegenwart von Natriumhydroxid in wäßrig alkoholischem Medium zum Sieden am Rückfluß erhitzt und dann in saurem Medium hydrolysiert zur Bildung der Säure der in Anspruch 1 definierten Formel III, welche Säure ein neues Produkt ist, das als Synthesezwischenprodukt zur Herstellung der Salze der in Anspruch 1 definierten Formel I gemäß den Ansprüchen 1 und 2 verwendet werden kann.

## Claims

**Claims for the following Contracting States : AT, BE, CH, DE, DK, FR, GB, IT, LI, LU, NL, SE**

1. The divalent metal salts of the acid 2-(N,N-di(carboxymethyl)amino) -3-cyano-4-carboxymethyl-5-carboxythiophene of the general formula I :

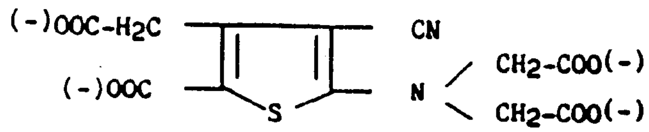

2 M (++)

(I)

wherein M represents a divalent metal such as strontium, calcium or magnesium.

2. The distrontium salt of the acid 2-(N,N-di(carboxymethyl)amino)-3-cyano-4-carboxymethyl-5-carboxythiophene in the form of the octahydrate, heptahydrate or tetrahydrate.

3. The acid 2-(N,N-di(carboxymethyl)amino)-3-cyano-4-carboxymethyl-5-carboxythiophene.

4. A process for the preparation of the divalent metal salts of claim 1, characterised in that there is used as starting material the tetraester of formula II :

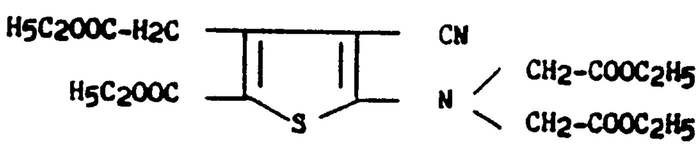

(II)

which is :

a) either heated at reflux in the presence of sodium hydroxide in an aqueous alcoholic medium, then hydrolysed in an acidic medium, to yield the acid of formula III :

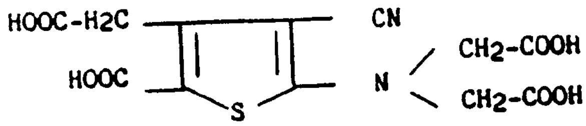

(III)

which is reacted, in aqueous medium, with a hydroxide of the general formula (IV) :

$$M(OH)_2 \quad (IV)$$

wherein M is as defined in claim 1;

b) or heated at reflux in a 50/50 mixture by volume of a normal solution of sodium hydroxide and ethanol to yield, after distilling off the solvents, the tetrasodium salt of formula V

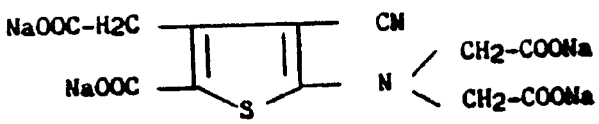

(V)

which is treated with an aqueous solution of a chloride of formula VI :

$$MCl_2 \quad (VI)$$

wherein M is as defined in claim 1;

c) or heated at reflux, in an aqueous alcoholic medium, with a hydroxide of the general formula IV defined above.

5. Pharmaceutical compositions comprising as active ingredient a salt of claim 1 together with appropriate pharmaceutical excipients.

6. Pharmaceutical compositions comprising as active ingredient the salt of claim 2 together with appropriate pharmaceutical excipients.

7. Pharmaceutical compositions according to claims 5 and 6 presented in a form suitable especially for the treatment of bone disorders, cutaneous and vascular ageing, hepatic disorders and dental disorders.

**Claims for the following Contracting State : GR**

1. A process for the preparation of the divalent metal salts of the acid 2-(N,N-di(carboxymethyl)amino)-3-cyano-4-carboxymethyl-5-carboxythiophene of the general formula I :

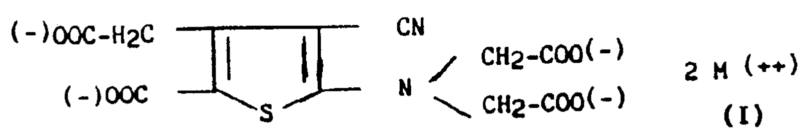

(I)

wherein M represents a divalent metal such as strontium, calcium or magnesium, characterised in that there is used as starting material the tetraester of formula II :

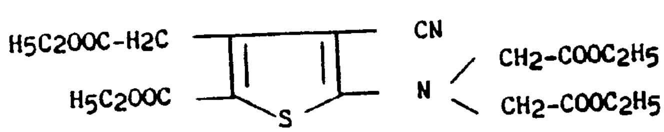

(II)

which is :

a) either heated at reflux in the presence of sodium hydroxide in an aqueous alcoholic medium, then hydrolysed in an acidic medium, to yield the acid of formula III :

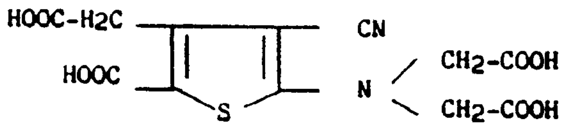

(III)

which is reacted, in aqueous medium, with a hydroxide of the general formula (IV) :

$M(OH)_2$ (IV)

wherein M is as defined above;

b) or heated at reflux in a 50/50 mixture by volume of a normal solution of sodium hydroxide and ethanol to yield, after distilling off the solvents, the tetrasodium salt of formula V

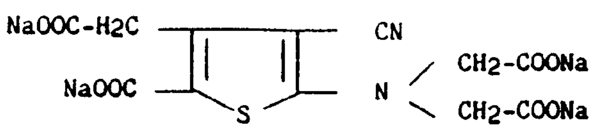

(V)

which is treated with an aqueous solution of a chloride of formula VI :

$MCl_2$ (VI)

wherein M is as defined above;

c) or heated at reflux, in an aqueous alcoholic medium, with a hydroxide of the general formula IV defined above.

2. A process according to claim 1, for the preparation of the distrontium salt of the acid 2-(N,N-di(carboxymethyl)amino) -3-cyano-4-carboxymethyl-5-carboxythiophene in the form of the octahydrate, heptahydrate or tetrahydrate.

3. A process for the preparation of the acid 2-(N,N-di(carboxymethyl)amino)-3-cyano-4-carboxymethyl-5-carboxythiophene, characterised in that : the tetraester of formula II defined in claim 1 is heated at reflux in the presence of sodium hydroxide in aqueous alcoholic medium, then hydrolysed in acidic medium, to yield the acid of formula III defined in claim 1, which acid is a new product used as a synthesis intermediate in the preparation, in accordance with claims 1 and 2, of the salts of formula I defined in claim 1.

**Claims for the following Contracting State : ES**

1. A process for the preparation of the divalent metal salts of the acid 2-(N,N-di(carboxymethyl)amino)-3-cyano-4-carboxymethyl-5-carboxythiophene of the general formula I :

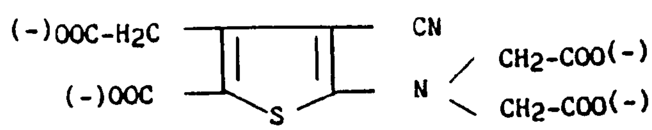

2 M (++)

(I)

wherein M represents a divalent metal such as strontium, calcium or magnesium, characterised in that there is used as starting material the tetraester of formula II :

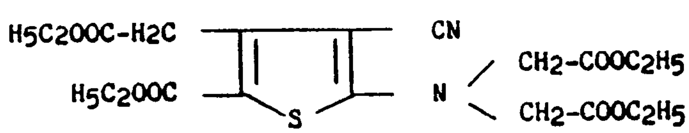

(II)

which is :

a) either heated at reflux in the presence of sodium hydroxide in an aqueous alcoholic medium, then hydrolysed in an acidic medium, to yield the acid of formula III :

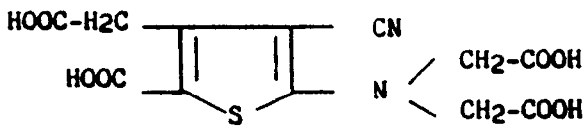

(III)

which is reacted, in aqueous medium, with a hydroxide of the general formula (IV) :

$$M(OH)_2 \quad (IV)$$

wherein M is as defined above;

b) or heated at reflux in a 50/50 mixture by volume of a normal solution of sodium hydroxide and ethanol to yield, after distilling off the solvents, the tetrasodium salt of formula V

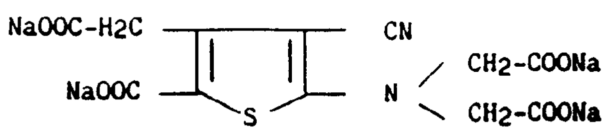

(V)

which is treated with an aqueous solution of a chloride formula VI :

$$MCl_2 \quad (VI)$$

wherein M is as defined above;

c) or heated at reflux, in an aqueous alcoholic medium, with a hydroxide of the general formula IV defined above.

**2.** A process according to claim 1, for the preparation of the distrontium salt of the acid 2-(N,N-di(carboxymethyl)amino)-3-cyano-4-carboxymethyl-5-carboxythiophene in the form of the octahydrate, heptahydrate or tetrahydrate.

**3.** A process for the preparation of the acid 2-(N,N-di(carboxymethyl)amino)-3-cyano-4-carboxymethyl-5-carboxythiophene, characterised in that : the tetraester of formula II defined in claim 1 is heated at reflux in the presence of sodium hydroxide in aqueous alcoholic medium, then hydrolysed in acidic medium, to yield the acid of formula III defined in claim 1, which acid is a new product used as a synthesis intermediate in the preparation, in accordance with claims 1 and 2, of the salts of formula I defined in claim 1.